# EUROPÄISCHE PATENTSCHRIFT

(11) **EP 0 456 237 B1**
(45) Veröffentlichungstag und Bekanntmachung des Hinweises auf die Patenterteilung: **05.08.1998**
(21) Anmeldenummer: 91107539.8
(22) Anmeldetag: 08.05.1991
(51) Int. Cl.: A61L 11/00

(54) **Fahrbare Desinfektionsanlage zur kontinuierlichen Desinfektion von Krankenhausmüll**
Mobile disinfection installation for continuous disinfecting of hospital waste
Installation mobile de désinfection pour la désinfection en continu d'ordures hospitalières

(30) Priorität: 09.05.1990 DE 4014856
(43) Veröffentlichungstag der Anmeldung: 13.11.1991
(73) Patentinhaber: Rilling, Siegfried, Dr., 72072 Tübingen (DE)
(72) Erfinder: Rilling, Eberhard, W-7000 Stuttgart 30 (DE)
(74) Vertreter: Riederer Freiherr von Paar zu Schönau, Anton

(56) Entgegenhaltungen:
- EP-A- 0 277 507
- DE-A- 2 950 638
- DE-C- 3 705 364
- DE-U- 8 816 225

## Beschreibung

Die Erfindung bezieht sich auf eine fahrbare Desinfektionsanlage zur kontinuierlichen Desinfektion von Krankenhausmüll nach dem Oberbegriff des Anspruchs 1.

Die Entsorgung von infektiösem Krankenhausmüll bereitet üblicherweise Probleme. Infektiöser Müll mit Spektrum "ABC" der Richtlinie des Bundesgesundheitsamts ist nach § 10a Abs. 1 BSeuchG zu vernichten. Dieser Müll umfaßt Wundverbände, blutige Tupfer, Kanülen, Lanzetten, Spatel, Einmalartikel wie Katheter, Spritzen, Uringefäße und Nierenschalen, Abfälle von Dialysestationen, Blutreste verfallener Blutkonserven, Einmal-Schutzkleidung, Einmal-Pflegeartikel aus Infektionsabteilungen und Hämodialyse, septische Operationsabfälle, Einmal-Laborgeräte wie Röhrchen, Pipetten usw. und ähnliches. Die Herkunft solchen Mülls ist nicht auf eigentliche Krankenhäuser beschränkt, er kommt auch aus ärtzlichen Labors, Blutbanken, Dialysestationen und sonstigen Einrichtungen, in denen mikrobiologisch gearbeitet wird.

Es gibt Bemühungen, derartigen Krankenhausmüll zu desinfizieren, im Volumen zu reduzieren und dann wie normalen Hausmüll zu entsorgen. Hierfür ist es erforderlich, vegetative bakterielle Keime einschließlich Mikrobakterien sowie Pilze einschließlich pilzlicher Sporen abzutöten, Viren zu inaktivieren, Sporen des Erregers des Milzbrandes abzutöten und Sporen der Erreger von Gasbrand, Gasödem und Wundstarrkrampf abzutöten.

Für diese Verarbeitung des Krankenhausmülls sind bereits zahlreiche Verfahren und Anlagen bekannt geworden, von denen die meisten stationär sind. Beispielsweise ist eine Anlage bekannt (DE-PS 35 05 570), bei der der Müll in einen senkrechten Aufnahmebehälter geschüttet und dort mit Wasser, dem etwas Desinfektionsmittel beigefügt sein kann, eingesprüht, anschließend zerschnitten und mit Hilfe eines Förderers, beispielsweise eines Schneckenförderers, unter Mikrowellenstrahlern hindurchgeschoben wird, die ihn aufgrund der eingesprühten Feuchtigkeit desinfizieren sollen. Bei einer anderen bekannten Anlage (DE-OS 25 05 185) wird der Müll in kontinuierlichem Betrieb in einer sich drehenden, rohrförmigen, schräg gelagerten Heißdampfkammer desinfiziert. Weiterhin ist eine Anlage bekannt (DE-PS 37 05 364), bei der aus einem senkrechten Einfüllbehälter das Material über ein Schneidwerkzeug zu einer mit Wärmeträgeröl erwärmten Schnecke gelangt, in deren Bereich eine Heißluft-Gegenströmung herrscht. Am Schneckenende befindet sich ein Lufteinlaß, am Schneckenanfang und auch im Einfülltrichter befinden sich Luft-Ansaugstellen.

Weiterhin sind mobile Desinfektionsanlagen bekannt. Hierbei ergeben sich jedoch stets Probleme mit der Baugröße. Beispielsweise ist eine Anlage zur nicht-kontinuierlichen Desinfektion bekannt (DE-OS 29 50 638), die als Nutzfahrzeugaufbau oder als fahrbarer Container ausgestaltet ist. Das Material wird in einen Autoklaven eingegeben, in den zur Desinfektion dann gespannter Dampf eingeleitet wird. Auch eine chemische Desinfektion ist möglich. Bei einer bekannten fahrbaren Desinfektionsanlage für kontinuierlichen Betrieb (DE-OS 38 00 821 = EP-A-0 277 507) erfordert die Baugröße einen Sattelschlepper-Anhänger oder -Auflieger. Von diesem Stand der Technik geht der Anspruch 1 in seinem Oberbegriff aus.

Der Erfindung liegt die Aufgabe zugrunde, auch bei für eine leichte Mobilität der Anlage geeigneter Baugröße zu einer kontinuierlichen vollkommenen Desinfektion des Krankenhausmülls zu kommen. Dies wird durch die im Anspruch 1 gekennzeichnete Erfindung gelöst. Der quaderförmige Nutzraum, innerhalb dessen die Desinfektionsanlage eingefügt ist, ist insbesondere der Nutzraum eines kastenförmigen Containers auf einer Wechselbrücke, wie sie für das Abstellen und Transportieren von Containern verwendet wird, er kann jedoch auch der kastenförmige Nutzraum eines LKW-Anhängers oder eines Lieferwagens sein. Durch die geschickte Einteilung der einzelnen Komponenten können diese in für die Funktion ausreichender Größe und mit Besonderheiten, die eine verkleinerte Baugröße ermöglichen, dicht gepackt werden, wobei im Nutzraum auch ein Filter unterbringbar ist, durch das sowohl aus dem infektiösen Bereich als auch aus dem desinfizierten Bereich Luft, Gas und Schwaden abgesaugt und gereinigt an die Umgebung abgegeben werden können. Insbesondere wird durch die Gasabsaugung im Bereich des Einfülltrichters eine gewisse Unempfindlichkeit gegenüber Undichtigkeiten geschaffen, die beispielsweise im Bereich des Trichterdeckels auftreten könnten, so daß hier eine einfachere, raumsparende Konstruktion zulässig ist. Der Aspiration des Einfülltrichters bedient im wesentlichen dann auch das Volumen des Schneckenförderers, wenn an dessen Abgabeende ein Zellenrad anschließt, über das der desinfizierte Müll ausgetragen wird. Dieses Zellenrad wirkt als Schleuse, die das Einströmen von Luft an dieser Stelle verhindert.

Die eigentliche Desinfektion findet entlang der Strecke des Schneckenförderers statt, die auf eine Temperatur von 140° aufgeheizt ist und so langsam angetrieben ist, daß die Verweilzeit der einzelnen Müllportion in dem Schneckenförderer etwa 15 Minuten beträgt. Die Schnecke ist sowohl mit Hilfe von Wärmeträgeröl als auch mit überhitztem Dampf geheizt, der aus Dampfdüsen ausströmt und den Müll gleichzeitig durchfeuchtet und aufgrund der Temperatur desinfiziert. Um die durch die kompakte Baugröße reduzierte Länge des Schneckenförderers voll ausnützen zu können, ist vorzugsweise nach Anspruch 9 in seinem Einlaufbereich, in den der noch nicht vorgewärmte Müll eintritt, noch eine Zusatzheizung vorhanden. Unmittelbar nach dem Schneidwerk können auch nach Anspruch 10 Wassersprühdüsen vorhanden sein, die dort den Müll anfeuchten und vorerwärmen.

Die Maßnahme nach Anspruch 2 macht von einem externen Wasseranschluß unabhängig, die Maßnahmen nach den Ansprüchen 3 und 4 ermöglichen den Betrieb mit einem Elektroanschluß mäßiger Leistung, wie er in vielen Fällen nur zur Verfügung steht.

Nach Abschluß des Betriebs muß die fahrbare Desinfektionsanlage zunächst eine Eigendesinfizierung durchführen, um dann wieder durch bewohntes Gebiet wegfahren zu können. Der Eigendesinfizierung dienen die konstruktiven Maßnahmen nach den Ansprüchen 11 und 12, wobei die Spritzdüsen mit heißem Wasser und/oder mit einem chemischen Desinfektionsmittel beliefert werden können. Bevorzugt wird die Eigendesinfektion mit Heißwasser, das Schmutzteile von der Oberfläche des Einfülltrichters und des Schneidwerks abwäscht und Bakterien abtötet. Wird ein chemisches Desinfektionsmittel gesprüht, so wird vorzugsweise ein biologisch abbaubares verwendet.

Der kontaminierte Krankenhausmüll wird in einen Einfülltrichter eingefüllt, von diesem über ein Schneidwerk einem unter desinfizierender Temperatur stehenden Schneckenförderer und weiterhin einem Bandförderer aufgegeben, der volumenreduzierten und desinfizierten Müll auswirft, der dann wie Hausmüll entsorgt werden kann. Durch eine sinnvolle Raumaufteilung im Nutzraum der Desinfektionsanlage lassen sich auf den sehr beschränkten zur Verfügung stehenden Raum die Komponenten der Desinfektionsanlage unterbringen, wobei aufgrund der hohen Leistungsdichte der einzelnen Komponenten die kompakte Anlage ein hervorragendes Ergebnis erbringt.

Weitere Einzelheiten, Vorteile und Weiterbildungen der Erfindung ergeben sich aus der folgenden Beschreibung eines bevorzugten Ausführungsbeispiels unter Bezugnahme auf die Zeichnung. Es zeigen:
Fig. 1 eine stirnseitige Ansicht einer erfindungsgemäßen Desinfektionsanlage auf einer Wechselbrücke mit untergesetztem Fahrgestell, wobei zur Darstellung einiger Innenteile die Stirnwand weggelassen ist;
Fig. 2 eine Draufsicht auf den Kasten der Desinfektionsanlage nach Fig. 1 mit abgenommener Deckenwand;
Fig. 3 in gewendeter Darstellung eine Schnittansicht in einer versetzten Ebene III-III in Fig. 2;

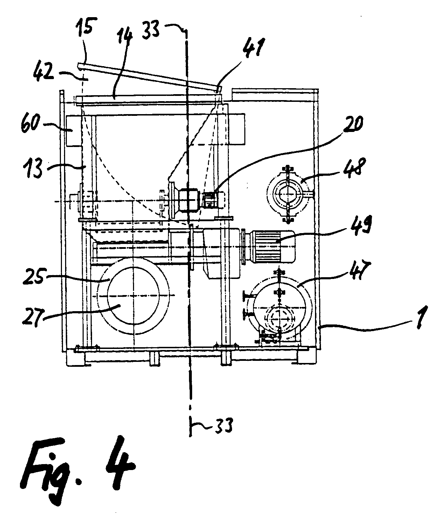

Fig. 4 eine Stirnansicht entsprechend Fig. 1 des Kastens der Desinfektionsanlage unter etwas vollständigerer Darstellung seiner Innenteile;

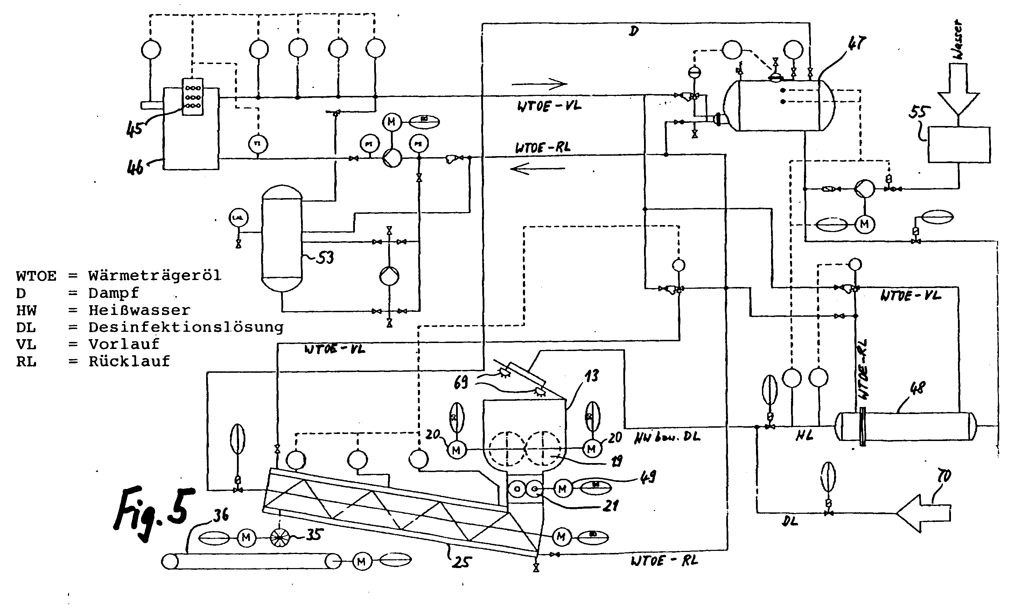

Fig. 5 ein Heiz- und Desinfektionsschema der Anlage;
Fig. 6 Innenansichten in Seitenansicht und Draufsicht einer in Einzelheiten abgewandelten Ausführung.

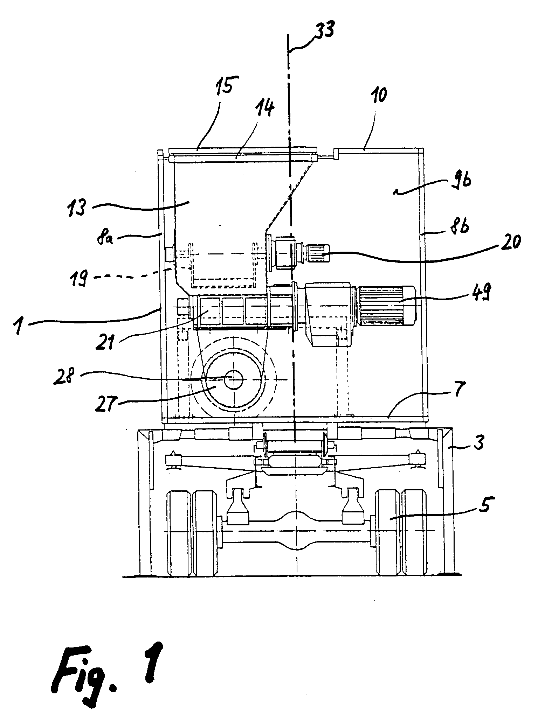

Die Desinfektionsanlage ist in einen kastenförmigen Container (1) einbezogen, der gemäß Fig. 1 auf einer Wechselbrücke (3) steht, der ein fahrbarer Untersatz (5) zum Anheben und Transportieren unterschoben werden kann. Der Container selbst hat eine Bodenwand (7), zwei längsseitige Seitenwände (8a) und (8b), zwei stirnseitige Seitenwände (9a) und (9b) und eine Deckenwand (10). An einem Ende des kastenförmigen Containers angrenzend an die stirnseitige Seitenwand (9a) mündet an der Deckenwand (10), und zwar in einer Ecke davon, ein Einfülltrichter (13) mit einer rechteckigen Mündungsfläche (14). Mit der Bezeichnung "Trichter" soll hier jede passende Aufnahmeform verstanden werden, beispielsweise kubisch, konisch und dergleichen. Die Mündungsfläche (14) ist so dimensioniert, daß auf sie, dicht abschließend, die Öffnungsfläche eines 1,1 m³-Standardmüllbehälters aufgesetzt werden kann. Sie ist ferner durch einen Klappdeckel (15) dicht verschließbar.

Im Einfülltrichter (13) befindet sich eine nach unten arbeitende Eindrückvorrichtung (19) in Form von zwei außermittig und gegenläufig rotierenden Drückflügeln, die jeweils von einem Elektromotor (20) angetrieben werden. Die Eindrückvorrichtung drückt eingeworfenen Müll auf ein Schneidwerk (21) zu. Zwischen der Eindrückvorrichtung und dem Schneidwerk oder hinter dem Schneidwerk ist eine (in der Zeichnung nicht sichtbare) Lichtschranke installiert, die bei Überfüllung an dieser Stelle abschaltet, bis dann stromabwärts wieder freier Raum entsteht.

An den Einfülltrichter schließt sich jenseits des Schneidwerks (21) als Desinfektionsstrecke ein Schneckenförderer (25) an, der im Bereich unter dem Einfülltrichter (13) von der Bodenwand (7) aus beginnt und parallel zu den längsseitigen Seitenwänden bis in die Nähe der gegenüberliegenden stirnseitigen Seitenwand (9b) verläuft, wobei er leicht aufwärts geneigt ist. Der Schneckenförderer (25) besteht aus einem Mantel (26) in Form eines Doppelwandmantels, der Heizeinrichtungen umschließt, und einer Schnecke (27) mit einer Hohlachse (28) und einem Schraubensteg (29). Der Schneckenförderer (25) liegt hinsichtlich einer Längs-Mittelebene (33) des kastenförmigen Containers (1) vollständig auf einer Seite dieser Längs-Mittelebene, also in derjenigen Querhälfte des kastenförmigen Containers (1), die durch die längsseitige Seitenwand (8b) begrenzt ist und der auch der größte Teil des Einfülltrichters (13) zugehört.

Im Bereich seines höher gelegenen, dem Einfülltrichter (13) abgewandten Endes weist der Mantel (26) des Schneckenförderers (25) eine Öffnung (34) auf, an die sich eine nach unten gehende Schleuse mit einem Zellenrad (35) anschließt, über das das Fördergut des Schneckenförderers aus diesem austragbar ist. Unterhalb des Zellenrades (35) befindet sich ein Bandförderer (36), der aus dem kastenförmigen Gehäuse aufgrund einer zu öffnenden Öffnungsklappe (37) ein Stück weit herausgezogen werden kann, wobei sein im Container befindliches Ende auch in der am weitesten herausgezogenen Stellung noch weiter innerhalb befindet als der Ausgang der Zellenradschleuse, während sein freies Ende bis zu einer passenden Übernahmestelle herausgezogen wird.

Der Bandförderer (36) liegt in seiner in den Container (1) eingeschobenen Stellung im wesentlichen unterhalb des schräg ansteigenden Schneckenförderers (25) und nimmt auch in der Breite nicht mehr als die Hälfte der Containerbreite ein. In diesem Bereich ist der Schneckenförderer (25) durch eine Stütze (38) in Form eines umgekehrten U abgestützt, unter der der Bandförderer (36) hindurchläuft.

Der Klappdeckel (15) des Einfülltrichters (13) ist an einer Achse (41) angelenkt, die in Längsrichtung des kastenförmigen Containers in der Nachbarschaft von dessen Längs-Mittelebene (33) verläuft. Seitlich am Deckel (15) sitzen Segmentbleche (42), die beim Schließen des Deckels in den Container (1) eintauchen und bei seinem Öffnen die Trichtermündung seitlich abschirmen. Eine in der Zeichnung nicht dargestellte Hub-Kipp-Vorrichtung hebt die Müllbehälter auf den kastenförmigen Container und setzt sie mit ihrer Mündungsfläche auf die geöffnete Mündungsfläche (14) auf.

Für die Arbeit der Desinfektionsanlage, also für das Durchschleusen des Krankenhausmülls unter gleichzeitiger Desinfektion und für die anschließende Eigendesinfektion der Anlage, stehen verschiedene Medien zur Verfügung, nämlich erhitztes Wärmeträgeröl, Dampf, heißes Wasser und chemisches Desinfektionsmittel. Als Energieträger sind Flüssiggas und ein Anschluß für elektrischen Strom vorhanden, weiterhin ist als Kontakt zur Außenluft eine Abluftfilteranlage eingebaut. Dies sei im folgenden ausführlicher dargelegt:

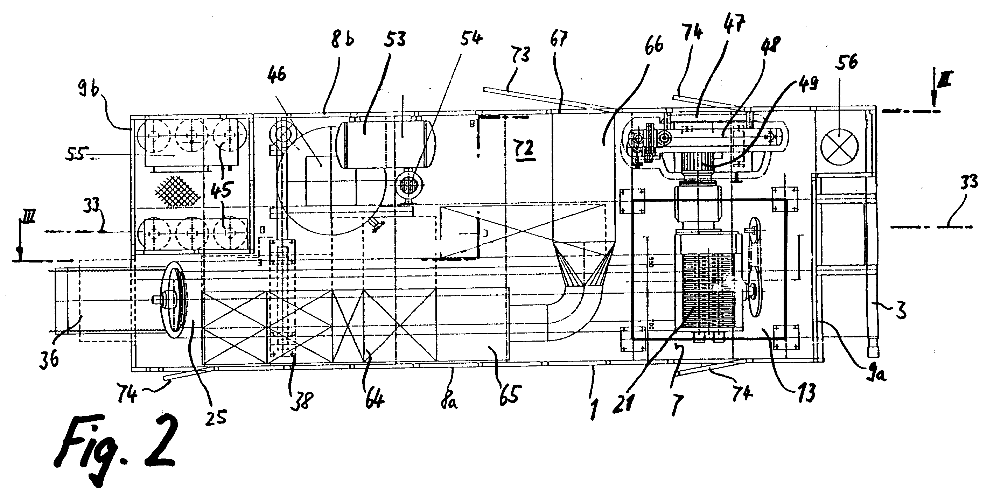

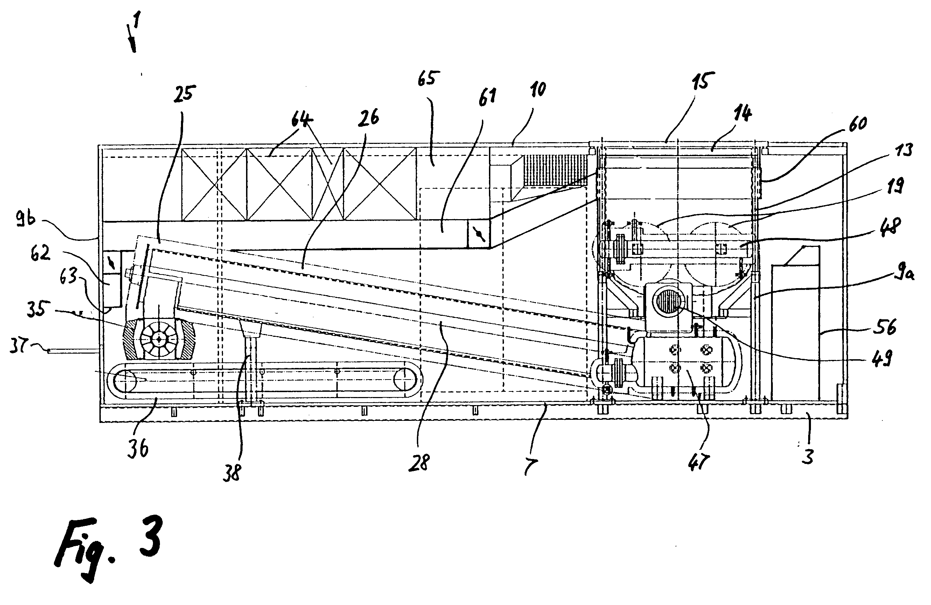

Ein Flüssiggasspeicher (45) befindet sich in der dem Einfülltrichter (13) diagonal gegenüberliegenden Position in der Ecke des kastenförmigen Containers (1) angrenzend an die Wände (8b) und (9b) und reicht hierbei im wesentlichen von der längsseitigen Seitenwand (8b) bis zur Längsmittelebene (33). Er faßt sechs Gasflaschen zu je 33 kg. An den Flüssiggasspeicher (35) schließt sich in Längsrichtung des Containers (1), wiederum in der durch den Schneckenförderer (25) nicht belegten Containerhälfte, ein Wärmeträgeröl-Heizofen (46) an, der flüssiggasbeheizt ist und eine Leistung von 100 kW umsetzt. Das im Heizofen (46) erhitzte Wärmeträgeröl verteilt sich nun auf drei verschiedene Verbraucher, nämlich einerseits den Schneckenförderer (25), der über seinen Mantel (25) Wärme aus dem Wärmeträgeröl-Kreislauf entnimmt, ferner einen Dampferzeuger (47), der in der nicht durch den Schneckenförderer (25) belegten Containerhälfte neben dem unteren Ende des Einfülltrichters (13) angeordnet ist, und einen Heißwassererzeuger (48), der in Form eines horizontal eingebauten Kessels über dem Dampferzeuger eingebaut ist. Ein Zwischenraum zwischen dem Dampferzeuger (47) und dem Heißwassererzeuger (48) ist bei der Ausführungsform nach den Fig. 2 und 3 durch einen elektrischen Antriebsmotor (49) mit angeflanschtem Getriebe für das Schneidwerk (21) und den Schneckenförderer (25) ausgenützt.

Oberhalb des Wärmeträgeröl-Heizofens (46) befinden sich noch ein Ausdehnungsgefäß (53) für den Wärmeträgeröl-Kreislauf und eine Umwälzpumpe (54). Oberhalb des Flüssiggasspeichers (45) befindet sich ein Kaltwasserspeicher (55). Beim dargestellten Ausführungsbeispiel ist in Verlängerung dieser der Medienbereitung dienenden, als "Servicehälfte" bezeichenbaren Hälfte des Containers (1) noch eine Wasserenthärtungsvorrichtung (56) angebracht.

Am Einfülltrichter (13) sitzt ein Absaugring (60), der an mehreren Stellen mit dem Trichterinneren kommuniziert und über eine Saugleitung (61) mit einer Saugluftleitung (62) verbunden ist. Die Saugluftleitung (62) besitzt eine Ansaugmündung (63) im Bereich der hinteren stirnseitigen Seitenwand (9b) über dem Bandförderer (36) und saugt Luft aus dem Innenraum des Containers (1) ab. Die Saugluftleitung (62) geht über in ein mehrstufiges Filter (64), einen Verdichter (65) und einen Schalldämpfer (66) bis zu einer Ausblasmündung (67). Beim dargestellten Beispiel besteht das Filter (64) aus einem Vorfilter EU4, einem Feinstfilter EU7, einem Schwebstoffilter S und einem Aktivkohlefilter A. Durch diese Filterkaskade wird die hindurchgeleitete Luft von Keimen und schädlichen Gasen gereinigt. Das Filter (64) und der Verdichter (65) liegen im Raum über dem Schneckenförderer (25), die Luftsaugleitung (62) erstreckt sich bis in den Bereich zwischen dem Schneckenförderer (25) und der benachbarten stirnseitigen Seitenwand (9b). Diese Teile liegen somit in der vom Schneckenförderer (25) belegten Containerhälfte. Aufgrund des Raumbedarfs des Filters und des Verdichters wird die Luftleitung indessen bei Annäherung an den Einfülltrichter (13) zur anderen Containerhälfte um 90° umgeleitet und geht dort in den Schalldämpfer (66) über, der sich bis zur Ausblasmündung (67) erstreckt, die einen Bereich entlang der oberen Kante der entsprechenden längsseitigen Seitenwand (8b) einnimmt.

Am Deckel (15) sitzen vier Sprühdüsenköpfe (69), die jeweils ihr Sprühgut in einem Raumwinkel von angenähert 360° abgeben. Sie sind mit einer Leitung verbunden, die alternativ mit dem Heißwassererzeuger (48) oder einem Desinfektionsmitteltank (70) verbindbar ist.

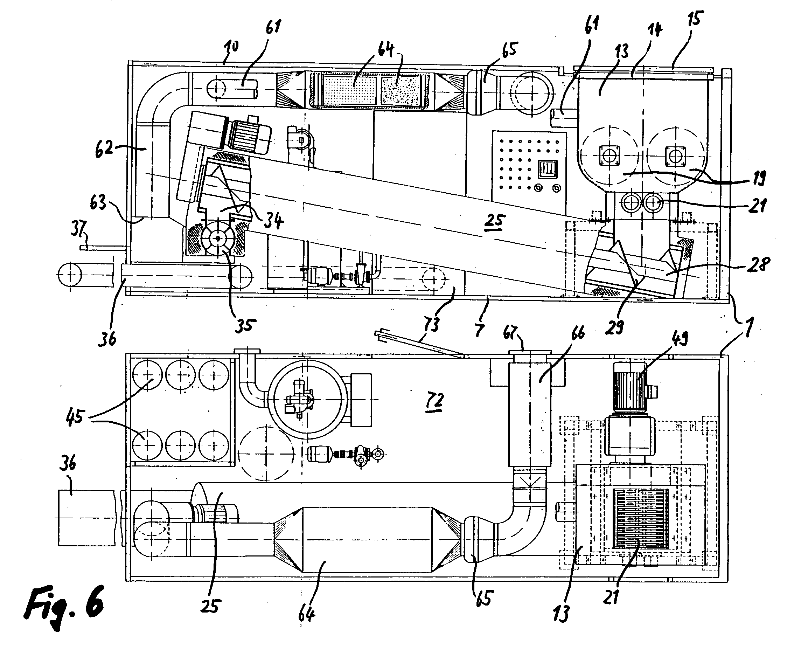

Die Anordnung der Einzelteile ist aus der Zeichnung klar erkennbar. In der Zeichnung sind weiterhin verschiedene elektromotorische Antriebe erkennbar, Fig. 6 zeigt einen alternativen Antrieb für den Schneckenförderer (25) in Form eines eigenen Antriebsmotors (71).

Zwischen dem Wärmeträgeröl-Heizofen (46) mit dem Ausdehnungsgefäß (43), dem Schalldämpfer (66), dem Heißwassererzeuger (78) und dem Dampferzeuger (77) befindet sich ein kleiner freier Raum (72), der von außen durch eine Klapp-Eingangstür (73) abgeschlossen ist. Von hier aus ist die Anlage für Zwecke der Kontrolle, Wartung und Reparatur betretbar. An anderen Stellen sind zum Zweck des Zugangs zu Baueinheiten weitere Montageöffnungen mit Klapptüren (74) vorhanden.

Die beschriebene Anordnung weist eine überaus kompakte Bauart auf. Trotzdem erfüllt sie sämtliche Erfordernisse sowohl hinsichtlich der einwandfreien Desinfektion als auch hinsichtlich der Möglichkeiten der Überwachung und Wartung. Das Heranbringen an den Einsatzort ist relativ problemlos.

Die Desinfektionsanlage arbeitet folgendermaßen:

Der kontaminierte Krankenhausmüll wird im Krankenhaus üblicherweise in Kunststoffsäcken von 0,15 mm Wandstärke sowie in Hartkunststoff-Containern mit 30 und 60 l Inhalt auf den Stationen gesammelt und bis zur Entsorgung bei Temperaturen unter 15°C in Müll-Großbehältern von 1,1 m³ Inhalt gelagert. Diese Behälter werden dann nach Eintreffen der fahrbaren Desinfektionsanlage mittels der Hub-Kipp-Vorrichtung auf die Mündungsfläche (14) des Einfülltrichters (13) gesetzt und in diesen Trichter entleert. Unmittelbar vor der Entleerung des Müllbehälters wird der Deckel (15) des Einfülltrichters (13) hydraulisch entriegelt und geöffnet. Mit Beginn der Öffnung des Deckels wird über den Absaugring (60), die Saugleitung (61), das Filter (64), den Verdichter (65) und den Schalldämpfer (66) Luft aus dem Einfülltrichter (13) abgesaugt und gereinigt durch die Ausblasmündung (67) an die Umgebung abgegeben. Durch diese Absaugung wird ein Austreten von kontaminierter Luft direkt in die Atmosphäre vermieden.

Nach der Entleerung des Müllbehälters in den Einfülltrichter (13) wird der Deckel (15) hydraulisch geschlossen und verriegelt. Mit dem Verriegeln des Deckels schaltet die Absaugvorrichtung automatisch um zum Zweck der zusätzlichen Absaugung der mit Dämpfen und Schwaden angereicherten Luft über dem Bandförderer (36) durch die Ansaugmündung (63). Außerdem wird durch die Absaugung eine Belüftung und Kühlung der Antriebsmotoren durchgeführt.

Im Einfülltrichter (13) wird nun der Müll, der im allgemeinen noch in den Kunststoffsäcken enthalten ist, durch die Eindrückvorrichtung (19) dem Schneidwerk (21) zugeführt. Durch das langsam laufende Schneidwerk wird der Müll auf eine Granulatgröße unter 20 mm fraktioniert und im Verhältnis von ca. 4:1 volumenreduziert. Es hat sich erwiesen, daß auch Metallgegenstände wie Spatel, Kanülen, Scheren usw., die im Müll enthalten sind, problemlos verarbeitet werden. Eine elektronische Reversiereinrichtung verhindert Beschädigungen des Schneidwerks und der Antriebsmotoren. Durch das Schneidwerk wird der Müll zerkleinert und dem Desinfektions-Schneckenförderer (25) kontinuierlich aufgegeben. Die im Einfülltrichter beim Schneidwerk (21) installierte Lichtschranke überwacht den Einschubvorgang und verhindert eine Überfüllung des Schneckenförderers (25) im Einlaufbereich. Sie bewirkt bei Überfüllung ein Anhalten der Eindrückvorrichtung, bis der überwachte Bereich wieder geräumt ist.

Der Schneckenförderer (25) wird in seinem Inneren durch das Wärmeträgeröl auf die Desinfektionstemperatur von 140°C erwärmt. Diese Temperatur wird durch Regelung konstant gehalten. Außerdem wird die Hohlachse (28) der Schnecke (27) mit Dampf beheizt, der außerdem an den dafür vorgesehenen Düsen in den Müll austritt. Der Müll wird somit angefeuchtet, erhitzt, umgewälzt und aufgelockert und während des Durchlaufs durch den Schneckenförderer (25) vollständig thermisch desinfiziert. Die Dampfeinleitung über die Hohlachse gibt gesättigten gespannten Wasserdampf ab. Die feuchte Temperatur von ca. 140°C und die Verweilzeit von ca. 15 Minuten genügen für die Desinfizierung. Am Ende des Schneckenförderers wird der volumenreduzierte und desinfizierte Müll über das Austrags-Zellenrad (35) dem Bandförderer (36) aufgegeben, der das resultierende Material einem krankenhauseigenen Preßcontainer zuführt, wo es eine weitere Volumenreduzierung erfährt und der Abfuhr als mikrobiologisch unbedenklich übergeben werden kann.

Aufsteigende Dampfschwaden über dem Bandförderer werden durch die Absaug- und Filtereinrichtung abgesaugt und gereinigt durch die Ausblasmündung (67) an die Umgebung abgegeben. Während des Betriebs wird außerdem der Einfülltrichter (13) ständig leicht abgesaugt, um sicherzustellen, daß über mögliche Leckstellen nicht kontaminierte Luft in die Umgebung oder in das Containerinnere entweichen kann. Aufgrund der Filterung werden keine die Umgebung beeinträchtigenden Gerüche oder Gase abgegeben. Wird ein Filterwechsel erforderlich, so werden die zu entsorgenden Filtermatten selbst in den Einfülltrichter (13) gegeben und in unbedenklichen Müll umgewandelt.

Nach Abschluß der Mülldesinfektion muß die Anlage eine Eigendesinfektion durchführen. Zur Eigendesinfektion steht heißes Wasser von mindestens 95°C zur Verfügung, das über die am Deckel (15) angeordneten vier Sprühdüsenköpfe (69) versprüht wird. Das heiße Wasser, das vom Heißwassererzeuger (48) kommt, reinigt aufgrund des Sprüh-Raumwinkels von 360° der Sprühdüsenköpfe sowohl die Deckel-Innenseite als auch den Einfülltrichter (13). Von den Oberflächen des Deckels (15), des Trichters (13) und des Schneidwerks (21) werden Schmutzteile abgewaschen und außerdem werden Bakterien abgetötet.

Die Desinfektionsanlage kann jedoch auch so betrieben werden, daß über die Sprühdüsenköpfe (69) ein chemisches Desinfektionsmittel, insbesondere eine biologisch abbaubare Desinfektionsmittel-Lösung, die vom Desinfektionsmitteltank (70) herangepumpt wird, eingesprüht wird. Die Einwirkungszeit beträgt etwa 30 Minuten. Sowohl bei Heißwasserbetrieb als auch bei chemischer Desinfektion wird während der Eigendesinfektion die Absaugeinrichtung weiter betrieben, so daß die Filteranlage entstehende Dämpfe absaugt und unschädlich macht.

Beim dargestellten Beispiel ist die Desinfektionsanlage mit einer elektrischen Regel-, Steuer- und Schaltanlage konzipiert, die eine sichere Selbstüberwachung der Anlage gewährleistet. Die Anlage kann nur im Automatik-Betrieb arbeiten, eine zweite Bedienebene, nämlich die Handebene, ist durch einen Schlüsselschalter gesichert. Sie ist nur für Wartung, Reparatur und Testzwecke vorgesehen. Der Austritt bedenklicher Materialien oder Gase in die Umgebung durch menschliche Fehlleistung ist damit im wesentlichen verhindert.

## Patentansprüche

1. Fahrbare Desinfektionsanlage zur kontinuierlichen Desinfektion von Krankenhausmüll, mit einem Einfülltrichter (13), dessen oberes Ende durch einen Deckel (15) oder einen aufgestülpten Müllbehälter dicht verschließbar ist, in dessen unterem Ende ein Schneidwerk (21) eingebaut ist und an dessen unterem Ende sich eine durch Wärmeübertragung mittels Wärmeträgeröl auf Desinfektionstemperatur aufheizbare Desinfektionsstrecke mit einem entlang seiner Länge dichten Schneckenförderer (25) anschließt, dessen Abgabeende über einem Bandförderer (36) liegt, der weiter unter einer mit einem Filter (64) in Verbindung stehenden Gasansaugmündung (63) hindurch zu einer Entladestelle läuft, dadurch gekennzeichnet, daß die Desinfektionsanlage kompakt in einen etwa quaderförmigen Nutzraum (1) eingefügt ist, an dessen Oberseite (10) sich im Bereich des einen längsseitigen Endes (9a) eine Öffnung (14) befindet, durch die das obere Ende des Trichters (13) zugänglich ist, und an dessen gegenüberliegender Endfläche (9b) eine mit einer Türe (37) versehene Öffnung vorhanden ist, durch die der Bandförderer (36) ausfahrbar ist, wobei der Schneckenförderer (25) mit nur kleinen Zwischenräumen zwischen sich und den endflächigen Stirnwänden (9a, 9b) den größten Teil der Längserstreckung des quaderförmigen Nutzraums in einer ersten der durch eine vertikale Längs-Mittelebene (33) getrennten Hälften des Nutzraums durchsetzt, während sich in der anderen, zweiten Hälfte des Nutzraums ein Energiedepot (45), eine Wärmeträgeröl-Heizeinrichtung (46) und eine Wasser-Heizeinrichtung (47, 48) befinden; daß der Schneckenförderer (25) außer der Heizung durch das erhitzte Wärmeträgeröl eine hohle Schneckenachse (28) enthält, die in den Raum des Schneckenförderers gerichtete Dampfdüsen aufweist und mit der Wasser-Heizeinrichtung (47) zur Dampfeinleitung verbunden ist, und daß eine Gas-Absaugleitung (60 bis 67) von der Gasansaugmündung (63) über das Filter (64) und einen Verdichter (65) zur Atmosphäre geleitet ist, wobei das Filter (64) sich in der ersten Hälfte des Nutzraums über dem Schneckenförderer (25) entlang seiner Deckenwand (10) hinzieht.

2. Desinfektionsanlage nach Anspruch 1, dadurch gekennzeichnet, daß sich in jener zweiten Hälfte auch ein Kaltwasserspeicher (55) befindet.

3. Desinfektionsanlage nach Anspruch 1 oder 2, dadurch gekennzeichnet, daß die Wärmeträger-Heizeinrichtung (46) und die Wasser-Heizeinrichtung (47, 48) ggf. in Kaskade zur Energiespeisung mit dem Energiedepot (45) verbunden sind und mechanische Antriebe (20, 49, 71) mit einer elektrischen Anschlußbuchse zur externen Energieeinspeisung verbunden sind.

4. Desinfektionsanlage nach Anspruch 3, dadurch gekennzeichnet, daß das Energiedepot (45) ein Lager für Flaschen mit Flüssiggas ist.

5. Desinfektionsanlage nach einem der Ansprüche 1 bis 4, dadurch gekennzeichnet, daß die Gas-Absaugleitung (60 bis 67) auch eine Gasansaugmündung im Einfülltrichter (13) aufweist.

6. Desinfektionsanlage nach einem der Ansprüche 1 bis 5, dadurch gekennzeichnet, daß in die Gas-Absaugleitung (60 bis 67) zwischen dem Filter (64) und der Mündung (67) zur offenen Atmosphäre ein Schalldämpfer (66) einbezogen ist.

7. Desinfektionsanlage nach Anspruch 6, dadurch gekennzeichnet, daß die im Bereich des hinteren Endes des Nutzraums (1) beginnende Filter-Verdichter-Schalldämpfer-Strecke der Gas-Absaugleitung (60 bis 67) sich in der Längsrichtung des Nutzraums in dessen erster Hälfte bis nahe an den Einfülltrichter (13) heranerstreckt, dort um 90° umgelenkt ist und quer durch die andere Hälfte des Nutzraums zur dort im oberen Bereich der Außenwand (8b) befindlichen Ausblasmündung (67) verläuft.

8. Desinfektionsanlage nach einem der Ansprüche 1 bis 7, dadurch gekennzeichnet, daß am Abgabeende des Schnekkenförderers (25) ein Zellenrad (35) anschließt, das auf den darunter durchlaufenden Bandförderer (36) entleert.

9. Desinfektionsanlage nach einem der Ansprüche 1 bis 8, dadurch gekennzeichnet, daß sich im Einlaufbereich des Schneckenförderers (25) eine zusätzliche Heizung in Form eines weiteren Wärmeträgeröl-Heizkreises befindet.

10. Desinfektionsanlage nach einem der Ansprüche 1 bis 9, gekennzeichnet durch Warmwassersprühdüsen im Bereich unmittelbar nach dem Schneidwerk.

11. Desinfektionsanlage nach einem der Ansprüche 1 bis 10, gekennzeichnet durch Sprühdüsenköpfe (69), die in den Einfülltrichter (13) gerichtet sind.

12. Desinfektionsanlage nach Anspruch 11, dadurch gekennzeichnet, daß die in den Einfülltrichter (13) gerichteten Sprühdüsenköpfe (69) an dessen Deckel (15) sitzen und mit der Wasser-Heizeinrichtung verbunden sind.

## Claims

1. Mobile disinfection installation for continuous disinfecting of hospital waste, with a hopper (13), whose upper end can be tightly closed by covering it with a lid (15) or by clapping a waste container on it, in whose lower end there is a set of cutters (21) and at whose lower end borders a disinfection line with a tight feed screw (25) along its length, which disinfection line can be heated up to the disinfection temperature by means of heat transfer via a thermal oil, whose discharge end is located above a belt conveyor (36) which continues below a gas intake opening (63) connected to a filter (64) and leads to an unloading site, characterized in that the disinfection installation is compactly incorporated in a more or less cuboid-shaped usefull room (1) at whose upper end (10) there is an opening (14) in the area of one of the longitudinal ends (9a), through which opening the upper end of the hopper (13) can be reached, and at whose opposite end surface (9b) there is a opening which is provided with a door (37) through which the belt conveyor (36) can move outwards, with the feed screw (25) with only small gaps between itself and the end faces (9a, 9b) interspersing the major part of the first half of the cuboid-shaped usefull room which is divided by a vertical longitudinal center plane (33), while the other, second half of the useful room contains an energy depot (45), a heat transfer oil heating system (46) and a water heating system (47, 48); that the feed screw (25) contains apart from the thermal oil heating system a hollow srew axle (28) which is provided with vapour nozzles directed into the room of the feed screw and which is connected to the water heating system (47) for introducing vapour and that a gas suction line (60 to 67) runs from the gas intake opening (63) via the filter (64) and a compressor (65) to the atmosphere, with the filter (64), in the first half of the useful room, running above the feed screw (25) along its ceiling (10).

2. Disinfection installation according to claim 1, characterized in that in said second half there is also a cold water reservoir (55).

3. Disinfection installation according to claim 1 or 2, characterized in that the heat transfer medium heating system (46) and the water heating system (47, 48) are, if connected, possibly in tandem, with the energy depot (45) in order to supply energy and that mechanical drives (20, 49, 71) are connected to an electrical socket for external energy supply.

4. Disinfection installation according to claim 3, characterized in that the energy depot (45) is a room for storing bottles containing liquified gas.

5. Disinfection installation according to any of claims 1 to 4, characterized in that the gas suction line (60 to 67) is also provided with a gas intake opening in the hopper (13).

6. Disinfection installation according to any of claims 1 to 5, characterized in that a silencer (66) is incorporated into the gas suction line (60 to 67) between the filter (64) and the opening (67) leading to the open atmosphere.

7. Disinfection installation according to claim 6, characterized in that the filter-compressor-silencer line of the gas suction line (60 to 67) beginning in the area of the rear end of the useful room (1) runs in the longitudinal direction of the first half of the useful room until close to the hopper (13), there it is diverted by 90° and runs across the other half of the useful room to the blow-out opening (67) which is there located in the upper area of the external wall (8b).

8. Disinfection installation according to any of claims 1 to 7, characterized in that at the discharge end of the feed screw (25) a bucket wheel (35) joins which drops its contents onto the belt conveyor (36) passing below.

9. Disinfection installation according to any of claims 1 to 8, characterized in that in the inlet area of the feed screw (25) there is an additional heating system in the form of another thermal oil heating circuit.

10. Disinfection installation according to any of claims 1 to 9, characterized by hot water spraying nozzles in the area immediately following the set of cutters.

11. Disinfection installation according to any of claims 1 to 10, characterized by spray nozzle holes (69) directed towards the inside of the hopper (13).

12. Disinfection installation according to claim 11, characterized in that the spray nozzle holes (69) directed towards the inside of the hopper (13) are attached to its lid (15) and connected to the water heating system.

## Revendications

1. Installation mobile de désinfection pour la désinfection en continu de déchets hospitaliers avec une trémie de chargement (13) dont l'extrémité supérieure peut être fermée de manière hermétique par un couvercle (15) ou par une poubelle renversée, à l'extrémité inférieure de laquelle est montée une déchiqueture (21) et à l'extrémité inférieure de laquelle se raccorde un parcours de désinfection chauffable à température de désinfection avec de l'huile caloporteur par transfert thermique avec un convoyeur à vis (25) étanche sur toute sa longueur, dont la sortie repose sur un convoyeur à bande (36), qui s'étend, sous une hotte d'aspiration de gaz (63) en liaison avec un filtre (64), jusqu'à un poste de déchargement, caractérisée en ce que l'installation de désinfection est installée de manière compacte dans un espace utile (1) approximativement parallélépipédique, à la face supérieure (10) duquel se trouve un orifice (14) dans le domaine d'une de ses extrémités longitudinales (9a), d'où on a accès à l'extrémité supérieure de la trémie (13), et à l'autre extrémité (9b) duquel existe un orifice muni d'une porte (37), par où peut sortir le convoyeur à bande (36) où le convoyeur à vis (25) passe avec seulement de faibles jeux entre lui et les parois frontales terminales (9a, 9b) dans la plus grande partie du prolongement longitudinal de l'espace utile parallélépidédique dans la première des moitiés de l'espace utile délimitées par un plan médian longitudinal (33), tandis que dans l'autre deuxième moitié de l'espace utile se trouve une réserve d'énergie (45), un dispositif de chauffage d'huile caloporteuse (46) et un dispositif de chauffage d'eau (47, 48); que le convoyeur à vis (25) en plus du chauffage par l'huile caloporteuse chauffée comprend un axe de vis creux (28), qui présente des buses de vapeur orientées dans la chambre du convoyeur à vis et est relié au dispositif de chauffage d'eau (47) pour introduire de la vapeur, et qu'une conduite d'aspiration de gaz (60 à 67) va de la hotte d'aspiration de gaz (63) via le filtre (64) et un compresseur (65) à l'atmosphère, où le filtre (64) s'étend dans la première moitié de l'espace utile au-dessus du convoyeur à vis (25) le long du plafond (10).

2. Installation de désinfection selon la revendication 1, caractérisée en ce que dans cette demi-moitié se trouve aussi un reversoir d'eau froide (55).

3. Installation de désinfection selon la revendication 1 ou 2, caractérisée en ce que le dispositif de chauffage de l'huile caloporteuse (46) et le dispositif de chauffage d'eau (47, 48) sont reliés le cas échéant en cascade pour l'alimentation en énergie à la réserve d'énergie (45) et les entraînements mécaniques (20, 49, 71) sont reliés à une prise électrique pour l'alimentation externe en énergie.

4. Installation de désinfection selon la revendication 3, caractérisée en ce que la réserve d'energie (45) est un stockage pour des bouteilles de gaz liquéfié.

5. Installation de désinfection selon l'une des revendications 1 à 4, caractérisée en ce que la conduite d'aspiration de gaz (60 à 67) présente aussi une hotte d'aspiration de gaz dans la trémie de déchargement (13).

6. Installation de désinfection selon l'une des revendications 1 à 5, caractérisée en ce qu' on dispose un silencieux (66) sur la conduite d'aspiration de gaz (69 à 67) entre le filtre (64) et le débouché (67) à l'atmosphère libre.

7. Installation de désinfection selon la revendication 6, caractérisée en ce que le parcours filtre-compresseur-silencieux de la conduite d'aspiration de gaz (60 à 67) débutant dans le domaine de l'extrémité arrière de l'espace utile (1), s'étend dans le sens longitudinal de l'espace utile dans sa première moitié jusqu'à la trémie de chargement (13), où elle fait un coude de 90° et traverse l'autre moitié de l'espace utile jusqu'à l'échappement de gaz (67) qui se trouve dans le domaine supérieur de la paroi externe (8b).

8. Installation de désinfection selon l'une des revendications 1 à 7, caractérisée en ce qu' à l'extrémité de sortie du convoyeur à vis (25) se raccorde une roue à godets (35) qui alimente le convoyeur à bande (36) qui s'étend en dessous.

9. Installation de désinfection selon l'une des revendications 1 à 8, caractérisée en ce que dans la domaine d'entrée du convoyeur à vis (25) se trouve un chauffage supplémentaire sous la forme d'un autre circuit de chauffage à huile caloporteuse.

10. Installation de désinfection selon l'une des revendications 1 à 9, caractérisée par les buses de pulvérisation d'eau chaude dans le domaine contigu à la déchiqueteuse.

11. Installation de désinfection selon l'une des revendications 1 à 10, caractérisée par des têtes de buses de pulvérisation (69) qui sont dirigées dans la trémie de chargement (13).

12. Installation de désinfection selon la revendication 11, caractérisée en ce que les têtes de buses de pulvérisation (69) dirigées dans la trémie de chargement (13) reposent sur son couvercle (15) et sont reliées au dispositif de chauffage d'eau.
